# EUROPEAN PATENT APPLICATION

(11) **EP 4 488 357 A1**
(43) Date of publication of application: **08.01.2025**
(21) Application number: 24187237.3
(22) Date of filing: 08.07.2024
(51) Int. Cl.: C12M 1/107, C12M 1/12

(54) **BIOREACTOR FOR PRODUCING BIOGAS**

(30) Priority: 07.07.2023 NO 20230771
(71) Applicant: High Performance Composites AS, 1640 Råde (NO)
(72) Inventor: Fediuk, Paal C., 1622 Gressvik (NO)
(74) Representative: Bryn Aarflot AS

(57) **Abstract**

A reactor for producing biogas from organic material, having a housing, and an assembly arranged inside the housing, the assembly being a combination of at least one perforated sheet and a plurality of random packing units. A method for maintaining a reactor including extracting the assembly from the housing, cleaning the assembly, and reintroducing the assembly into the housing.

## Description

### INTRODUCTION

The present invention concerns a reactor for the production of biogas from organic material. The reactor may be used e.g. in the production of biogas such as methane from organic waste such as manure or muck. The reactor may be used for the extraction of a variety of gases for remediation of organic waste or capture of gases e.g. carbon capture or ammonia capture. The invention further relates to a method for maintaining a reactor.

### BACKGROUND

The bacteria present in the organic waste are going through biochemical processes that produce gasses, commonly ammonia (NH₃), carbon dioxide (CO₂), methane (CH₄), and hydrogen sulfide (H₂S). The production of biogas from organic waste inside reactors offers a way to extract and produce gas from material that would not otherwise be utilized. Furthermore, in case this material is left untreated, all these gases would end up in the atmosphere, contributing to the greenhouse effect or in general worsening the air quality.

Biomass and Bioenergy Volume 161, June 2022, 106446 discloses different bioreactor configurations for biogas production from sugarcane vinasse.

JP2008037903A discloses a device for producing the biogas by performing the methane fermentation by using the fishery process bio-materials as the main raw materials is equipped with a fermentation vessel of outside circulation type, and an immobilizing carrier of the methane fermentation bacterial group housed in the fermentation vessel. The immobilizing carrier consists of an immobilized bed by arranging a multiple number of plate-formed non-woven fabrics as inclined, along the circulation direction of the methane fermentation raw materials including the fishery process waste materials.

JP2004041929A discloses immobilized microorganisms packed in a methane fermentation tank are composed of a material in which anaerobic microorganisms mainly consisting of methane fungi are supported on a carrier formed by knitting a synthetic resin wire rod in the shape of a string, a carrier of a synthetic resin polyhedron having the largest diameter or one side of 5-20 mm length or granules, or a biodegradable plastic carrier. The carrier is preferably hydrophilicized by an oxidizing agent, and the oxidizing agent is preferably ozone gas. Preferably, the organic waste is circulated in the methane fermentation tank, a gas produced by the methane fermentation is introduced into the organic waste in the tank, and the waste is methane-fermented while being agitated mechanically, for example, with agitation blades.

US5563069A discloses an apparatus and method for converting organic materials such as sugars and acids into other organic materials such as organic acids and salts other than the starting materials with immobilized cells. The invention is applicable to the conversion of the lactose content of whey, whey permeate or other lactose containing solutions and wastes into lactic acid, propionic acid, acetic acid, and their salts. The fermentation cells may be homolactic, homoacetic and propionic bacteria. The cells are immobilized onto the surface of and within convoluted sheets of a fibrous support material and reactant bearing fluids are caused to flow between the opposing surfaces of such convoluted sheets. Lactose containing solutions such as whey and whey permeate may be co-fermented with homolactic and homoacetic bacteria to acetic acid or acetate. The product may be extracted from its aqueous media by high distribution coefficient solvents particularly trioctylphosphine oxide and long-chain aliphatic secondary, tertiary and quaternary amines. The process and apparatus are particularly amenable to the economical production of calcium magnesium acetate and potassium acetate, which are useful as road deicing and anti-icing agents. The process and apparatus are also amenable to the economical production of calcium propionate and sodium lactate, which are useful as food preservatives.

However, the above-mentioned documents use primarily fixed bedding.

The biochemical reactions of bacteria producing biogases depend on many parameters such as temperature, humidity, pressure and available reacting surface, to name a few non-limiting examples. In order to achieve high yields of gases, usually reactors are space-consuming. Additionally, the cleaning and maintenance of larger devices is more demanding and time-consuming.

The presence of solids in the material can make the maintenance of a reactor demanding and unpractical, since the increased surface area increases the chances of potential blockings from solids.

The invention provides an apparatus and method which alleviates the above-mentioned problems, offering a solution for improved gas yield efficiency and easier device maintenance.

The invention provides a reactor for producing biogas from organic material, and more specifically manure, a type of organic material in solid form, or in liquid form containing solids.

### SUMMARY OF THE INVENTION

The invention relates to a reactor for producing biogas from organic material, such as manure, comprising a housing, and an assembly arranged to be incorporated inside the housing.

The assembly may be adapted to increase a surface area inside the housing and allow unobstructed passage along at least one direction. The assembly may be made of fiberglass material or of plastic material. The assembly may be a spirally-formed plate or a crenulated-formed plate. The assembly may further be at least one perforated sheet, a plurality of random packing units, or a combination of an at least one perforated sheet and a plurality of random packing units. The plurality of random packing units may be arranged on top of the at least one perforated sheet. The at least one perforated sheet may be corrugated.

The at least one perforated sheet may comprise a plurality of perforated sheets, said plurality of perforated sheets being arranged to create an enclosure for the plurality of random packing units to be disposed within. The plurality of sheets may be further arranged to create a plurality of enclosures, each enclosure having a fraction of the plurality of random packing units disposed within them.

An inner surface of the housing may be made of fiberglass material. The reactor may further comprise a substrate inlet, a substrate outlet, a gas outlet and isolation arranged on the housing.

The invention further relates to a method for maintaining a reactor according to comprising extracting from a housing an assembly, cleaning the assembly, and reintroducing the assembly into the housing. The method may further comprise cleaning the housing, after extracting at least one perforated sheet with a plurality of random packing units.

The invention further relates to a use of a reactor for the production of biogas from organic material, and for the extraction of gases from organic material for remediation of organic waste or capture of gases.

The principle behind the invention is to increase the effective surface on which the bacteria grow, and thereby increase the amount of produced gas per unit of volume. This results in a more compact and space-efficient reactor design.

The invention further offers an apparatus and a method for achieve easier management of the assembly when it comes to cleaning or maintenance.

### BRIEF DESCRIPTION OF DRAWINGS

Examples of the invention are disclosed with reference to the following drawings, wherein:
Fig. 1 shows an exemplary schematic of a reactor;
Fig. 1a shows a top view of a spirally formed plate;
Fig. 1b shows an exemplary triangular shaped spirally formed plate;
Fig. 1c shows an exemplary oval shaped spirally formed plate;
Fig. 1d shows an exemplary perpendicular shaped spirally formed plate;
Fig. 2 shows an exemplary crenulated-formed plate;
Fig. 3a shows an exemplary assembly being a perforated sheet;
Fig. 3b shows an exemplary reactor with the assembly being a perforated sheet;
Fig. 4a shows an exemplary reactor with the assembly being a plurality of random packing units;
Fig. 4b shows an exemplary assembly being a random packing unit;
Fig. 5a shows an exemplary reactor where the assembly is a combination of at least one perforated sheet and a plurality of random packing units;
Fig. 5b shows an exemplary reactor where a plurality of perforated sheets is arranged to create an enclosure for the plurality of random packing units to be disposed within;
Fig. 5c shows an exemplary perforated sheet;
Fig. 6a shows an exemplary reactor where the assembly is a combination of at least one corrugated perforated sheet and a plurality of random packing units; and
Fig. 6b shows an exemplary reactor where a plurality of corrugated perforated sheets is arranged to create an enclosure for the plurality of random packing units to be disposed within.

### DETAILED DESCRIPTION

Example embodiments are described with reference to the drawings. The examples are illustrations only and are not limiting for the invention.

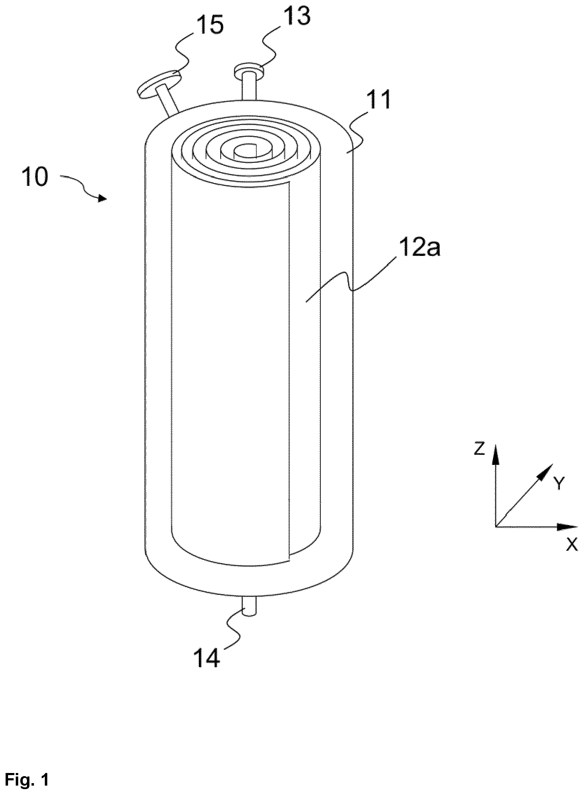

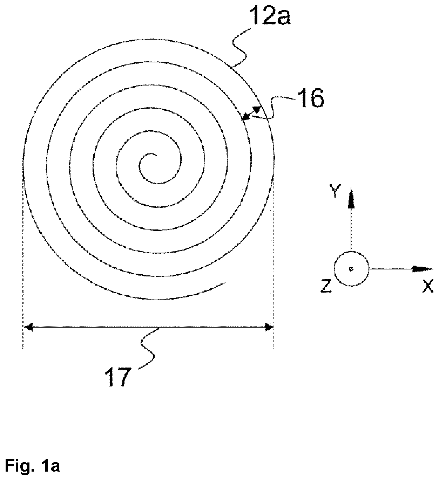

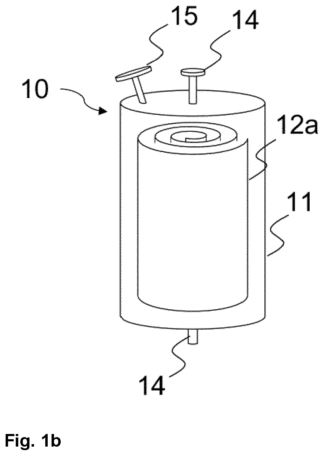

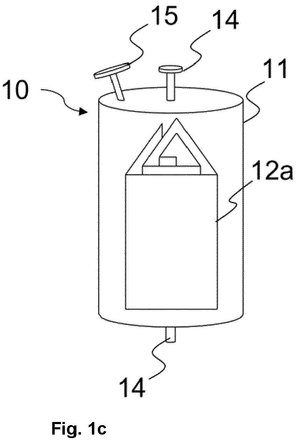

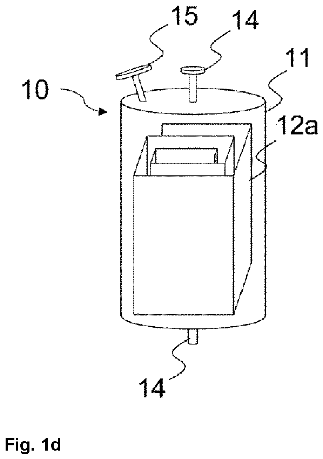

Fig. 1 shows an exemplary schematic of a reactor 10 for producing biogas from organic material. The reactor comprises a housing 11. The reactor further comprises an assembly 12a arranged to be incorporated inside the housing 11. The housing 11 has an inner surface. The inner surface of the housing is made of fiberglass material. The reactor further comprises isolation (not shown). The isolation may be arranged on the exterior of the housing 11 and it may be of glass wool material. The isolation is used for reducing or eliminating the leak of gasses out of the reactor and to better control the conditions inside the reactor. Bacteria grow inside the reactor and produce a variety of gasses which are collected. The optimal growth temperature of the bacteria depends heavily on the type of bacteria.

In Fig. 1, the reactor 10 further comprises a substrate inlet 13 and a substrate outlet 14 arranged on the housing. The substrate inlet 13 and substrate outlet 14 are adapted to control the flow of substrate material in to and out of the reactor 10, respectively.

The reactor 10 further comprises a gas outlet 15 arranged on the housing 11. The gas outlet 15 is adapted to extract produced gas out of the reactor.

The organic material enters the bioreactor 10 from the substrate inlet 13 at a top end if the reactor in the in-use orientation, passes through assembly 12a and exits the bioreactor 10 from a substrate outlet 14 in the bottom. The substrate material may be organic waste such as manure or muck, in solid or fluid form. This substrate material may be mixed with fluids, such as water, or solids in order to achieve optimal viscosity and optimize the passage through the assembly 12a. The assembly 12a is adapted to allow unobstructed passage along at least one direction.

The assembly 12a has an increased surface area which increases the available surface on which the bacteria can grow on. The assembly 12a is adapted to increase a surface area inside the housing. The larger the available surface, the larger the amount of bacteria that can be cultivated upon the surface within the reactor, which in turn leads to higher gas yields. As the bacteria grow on the surface of the assembly 12a, they produce a variety of gasses which are collected from the gas outlet 15.

With further reference to Fig. 1, the assembly 12a is a spirally-formed plate 12a. The spirally-formed plate 12a in Fig. 1 is a plate that is rolled around a vertical z-axis to form a cylindrical column with a circular cross-section. A top view of the spirally-formed plate 12a is shown in Fig. 1a. A distance between each spiral plate loop 16 allows the organic material to pass unobstructed in the z-direction between each loop. The spirally formed plate 12a has a diameter 17.

The spirally-formed plate 12a may also have different cross-sectional geometry than circular shown in Fig. 1a. It may be for example triangular, oval shaped or perpendicular, as shown in Fig. 1b, 1c, and 1d. The principle behind the spirally-formed plate 12a is to better utilize the volume of the housing of the reactor using the increased effective surface area of the plate. At the same time the spirally-formed shape allows for unobstructed passage along a direction of choice, z-direction in the case of Fig. 1, between each loop.

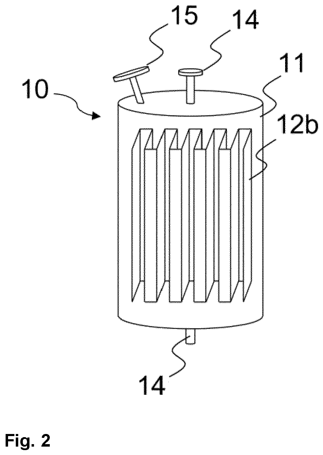

The assembly 12 may also be a crenulated-formed plate 12b as shown in Fig. 2. The crenulated-formed plate 12b may also utilize better the volume of the housing of the reactor by using the increased effective surface area of the plate, while allowing for unobstructed passage along a direction of choice.

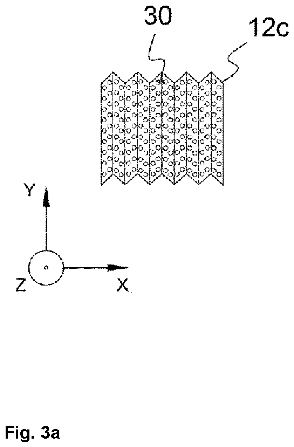

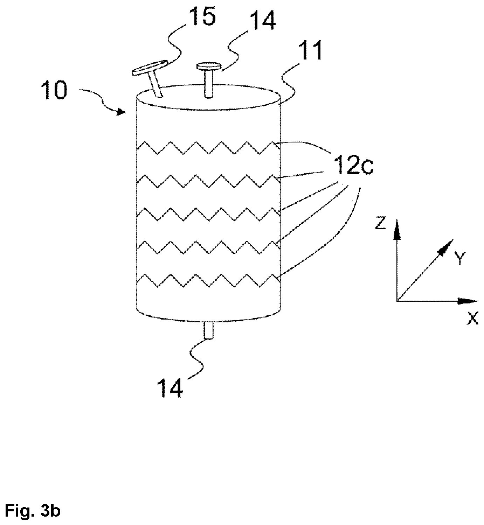

An alternative exemplary assembly 12 is shown in Fig. 3a, wherein the assembly is at least one perforated sheet 12c. A type of perforation may be holes 30. The perforation makes the sheet permeable. The at least one perforated sheet 12c is corrugated. The corrugation further contributes to the increase of the effective surface area of the assembly. Each at least one perforated sheet 12c may be placed at various heights (i.e. along the z-axis as per the axis in Fig. 3b) within the housing 11, creating a layered structure within the housing as shown in Fig. 3b. The perforated sheets may match the horizontal inner cross section geometry of the housing in order to compartmentalize the housing without leaving substantial gaps.

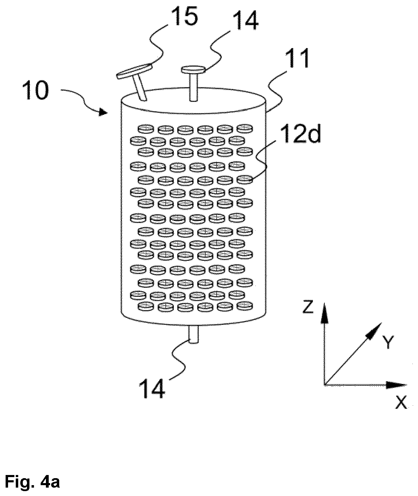

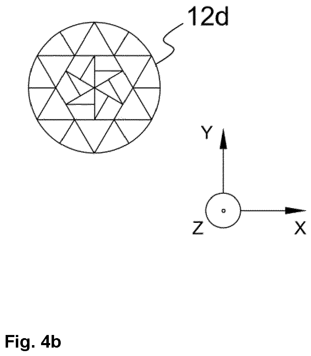

Another exemplary assembly 12 is shown in Fig. 4a, wherein the assembly is a plurality of random packing units 12d. Each random packing unit is permeable allowing material to pass through its circumference. Each random packing unit has a pattern which increases the surface area of each unit. In Fig. 4b, the random packing unit 12d is a disc with a perforated pattern within its circumference. The plurality of random packing units is disposed within the housing as shown in Fig. 4a, as a random assembly. Each packing unit 12d offers an unobstructed passage of substrate material through the holes in its local z-direction.

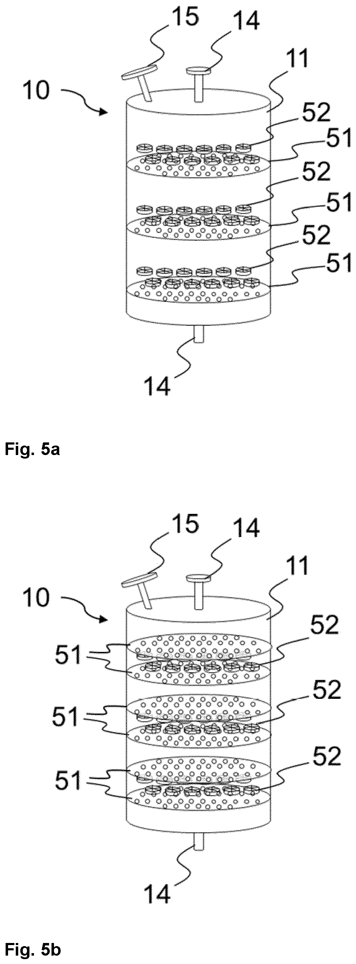

Fig. 5a shows another exemplary assembly, wherein the assembly is a combination of at least one perforated sheet 51 and a plurality of random packing units 52. The plurality of random packing units 52 is arranged on top of the at least one perforated sheet 51. The assembly of 5a can use the at least one perforated sheet as a support structure for a plurality of packing units to be disposed upon.

The assembly may occupy a percentage of the available internal volume of the housing, that being the effective volume.

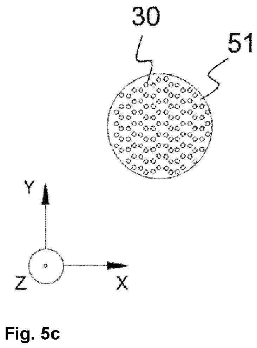

With reference to Fig. 5b, a plurality of perforated sheets 51 can be used to create an enclosure within which packing units 52 may be disposed. The plurality of sheets are arranged to create a plurality of enclosures, each enclosure having a fraction of the plurality of random packing units 52 disposed within them. The sum of each fraction of the plurality of random packing units 52 disposed within each plurality of enclosures adds up to the plurality of random packing units 52. The area between each enclosure may be further filled with packing units to further utilize the volume. Alternatively, it may be left empty for providing a volume for organic material or gas from the organic material to circulate. Some or all of the packing units in the plurality of packing units may be the packing unit 12d shown in Fig. 4b. Fig. 5c shows an example perforated sheet 51 having holes 30. Some or all of the perforated sheets 51 may be the example perforated sheet shown in Fig. 5c.

Both the packing units and the perforated sheet are permeable. Hence, material can move unobstructed along each individual random packing unit and each perforated sheet.

At the same time, the packing units can be localized upon the sheet or within an enclosure made of sheets. This offers easier management of the assembly 12 when it comes to cleaning or maintenance, because the packing units are constrained on top of the perforated sheet or within the enclosure of perforated sheets, thereby making it easier to move them in and out of the housing 11.

An exemplary method for maintaining a reactor comprise extracting the assembly from the housing. The method further comprises cleaning the assembly. After the assembly is cleaned, it is reintroduced into the housing. As long as the assembly is out of the housing, there is the possibility for cleaning of the housing.

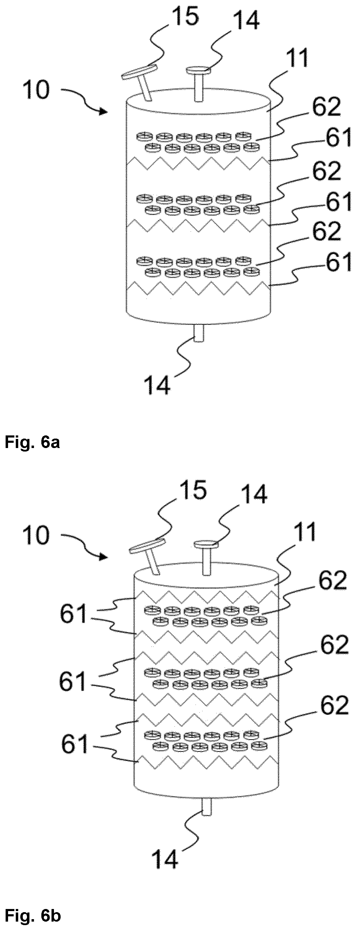

Fig. 6a shows another exemplary assembly, wherein the assembly is a combination of at least one perforated sheet 61, wherein the at least one perforated sheet is corrugated, and a plurality of random packing units 62. The perforated corrugated sheet may be of the geometry shown in Fig. 3a or Fig. 5c. The specific examples in Figs. 6a and 6b with the corrugated perforated sheets have the same benefits as the non-corrugated example, that being unobstructed along each individual random packing unit and each perforated corrugated sheet, and the easier management of the assembly 12 when it comes to cleaning or maintenance. This is because the packing units are constrained on top of the perforated sheet or within the enclosure of perforated sheets, thereby making it easier to move them in and out of the housing 11. In addition, the corrugation may further increase the surface area of the sheet, and diversify the amount of volume the gas from the organic material has available.

The assembly 12 may be made of fiberglass material. The assembly 12 may be made of plastic material. Both fiberglass and plastic material offer low cost and versatility in terms of manufacturing.

The reactor may be used for the production of biogas from organic material, such as manure or muck. Furthermore, the reactor may be used for the extraction of gases from organic material, such as manure or muck, for remediation of organic waste or capture of gases.

The use of the examples below should not be considered limiting for the invention, but are only intended for explanation purposes. Other configurations than the ones presented may be envisaged depending on the system design and use.

### Example 1

This is an example only illustrating the concept and is not limiting for the invention.

In the example of Fig. 1, the diameter 17 of the spirally formed plate is 2,85 m. Between each spiral loop there is a distance 16 of 0,05 m. If we assume that the height of the spirally formed plate column is 10 m this leads to a surface area of 1.230 m² on each side, totalling 2.460 m² on both sides. For a cylindrical surface of the same diameter and height, the equivalent surface area would be ≈ 895 m² on each side, totalling 1.790 m² on both sides. This results in an approximately 37% increased surface area for the same volume.

### Example 2

This is an example only illustrating the concept and is not limiting for the invention.

In the example of Fig. 4a, each packing unit 12d has a surface area of 0,017245 m². Its diameter is 0,093 m and its thickness 0,030 m. This gives a volume of approximately 0.0002 m³ for each packing unit. If we assume that the reactor has an effective volume of 100 m³ allocated for the assembly, then in this effective volume would fit 500.000 packing units, resulting in a surface area of approximately 8.623 m².

### Example 3

This is an example only illustrating the concept and is not limiting for the invention.

In the example of Fig. 5a, a plurality of packing units is disposed on top of a perforated sheet. Each packing unit has a surface area of 0,017245 m². The diameter of each packing unit is 0,093 m and its thickness 0,030 m. This gives a volume of approximately 0,0002 m³ for each packing unit.

Each side of a perforated sheet will have a surface area of more than 0,17 m², based on the amount, distribution and size of perforations etc. The perforated sheet has a length of 0,536 m, a width of 0,317 m and a height of 0,026 m. Therefore 0,17 m² x 2 (for each side) = 0,34 m² would be the minimum surface area of a perforated sheet of these dimensions. The volume of each perforated sheet is approximately 0,0044 m³. Therefore, for each perforated sheet used, 22 packing units need to be extracted in terms of volume, based on their relative volumes.

If three perforated sheets are used, 0,0132 m³ need to be substituted, hence 66 packing units.

The total surface area for such a reactor would then be approximately 8622 m²: 499934 packing units x 0,017245 m² **≈** 8.621 **m²**
plus
3 perforated sheets x 0,34 m² **(minimum) ≈ 1 m²** (minimum)

If we compare this number with the result from example 2 above, where only packing units were used in the housing, the introduction of perforated sheets does not change that much the effective surface area inside the housing for the same effective volume.

More specifically for that example, for each 22 substituted packing units with one perforated sheet of the above dimensions, there is a loss of surface area of approximately 0,04 m², since 22 packing units have a surface area 0,37939 m² and each sheet 0,34 m². This surface area loss may be considered minimal compared to the overall surface area, which is 5 orders of magnitude larger.

Hence, there is a combined effect, since the incorporation of perforated sheets can assist in the management of the cleaning and maintenance of the reactor, whilst allowing for an arrangement in which an available surface area within the reactor is significantly increased.

Having described example embodiments of the invention it will be apparent to those skilled in the art that other embodiments incorporating the concepts may be used. These and other non-limiting examples illustrated above are intended by way of example only and the actual scope of the invention is to be determined from the following claims.

## Claims

1. A reactor for producing biogas from organic material, comprising:
a housing, and
an assembly arranged inside the housing, the assembly being a combination of at least one perforated sheet and a plurality of random packing units.

2. The reactor according to claim 1, wherein the assembly is made of plastic material.

3. The reactor according to claim 1 or claim 2, wherein the plurality of random packing units is arranged on top of the at least one perforated sheet.

4. The reactor according to any one of claims 1 - 3, wherein the at least one perforated sheet is corrugated.

5. The reactor according to any one of claims 1 - 4, wherein the at least one perforated sheet comprises a plurality of perforated sheets, said plurality of perforated sheets arranged to create an enclosure for the plurality of random packing units to be disposed within.

6. The reactor according to claim 5, wherein the plurality of sheets is further arranged to create a plurality of enclosures, each enclosure having a fraction of the plurality of random packing units disposed within them.

7. The reactor according to any one of claims 1 - 6, wherein an inner surface of the housing is made of fiberglass material.

8. The reactor according to any one of claims 1 - 7, further comprising:
a substrate inlet, and a substrate outlet, arranged on the housing.

9. The reactor according to any one of claims 1 - 8, further comprising:
a gas outlet, arranged on the housing.

10. The reactor according to any one of claims 1 - 9, further comprising:
isolation, arranged on the housing.

11. A method for maintaining a reactor according to any one of claims 1 - 10, comprising:
extracting the assembly from the housing;
cleaning the assembly; and
reintroducing the assembly into the housing.

12. The method of claim 11, further comprising:
cleaning the housing, after extracting the assembly.

13. Use of a reactor according to any one of claims 1 - 10 for the production of biogas from organic material.

14. Use of a reactor according to any one of claims 1 - 10 for the extraction of gases from organic material for remediation of organic waste or capture of gases.
